# EUROPEAN PATENT SPECIFICATION

(11) **EP 4 028 768 B1**
(45) Date of publication and mention of the grant of the patent: **07.08.2024**
(21) Application number: 20767850.9
(22) Date of filing: 11.09.2020
(51) Int. Cl.: G01N 33/558, G01N 33/569

(54) **LATERAL FLOW IMMUNOASSAY DEVICE FOR DETECTION OF CANDIDA INFECTION AND USES THEREOF**
IMMUNOASSAY-VORRICHTUNG MIT LATERALEM DURCHFLUSS ZUM NACHWEIS VON CANDIDA-INFEKTION UND VERWENDUNGEN DAVON
DISPOSITIF DE DOSAGE IMMUNOLOGIQUE À ÉCOULEMENT LATÉRAL POUR LA DÉTECTION D'INFECTION PAR CANDIDA ET SES UTILISATIONS

(30) Priority: 13.09.2019 EP 19197213
(43) Date of publication of application: 20.07.2022
(73) Proprietor: GaDia SA, 1870 Monthey (CH)
(72) Inventor: DUCREST, Percevent, 1892 Lavey (CH)
(74) Representative: reuteler & cie SA
(86) International application number: PCT/EP2020/075462
(87) International publication number: WO 2021/048345

(56) References cited:
- WO-A1-2014/001574
- ZHENG-XIN HE ET AL: "Development of a Lateral Flow Immunoassay for the Rapid Diagnosis of Invasive Candidiasis", FRONTIERS IN MICROBIOLOGY, vol. 7, 13 September 2016 (2016-09-13), XP055673697, DOI: 10.3389/fmicb.2016.01451

## Description

### Field of the invention

The present invention relates to a method for the detection of *Candida spp.* infection in a sample by lateral flow immunoassay and a lateral flow immunoassay device, in particular useful for the early stage detection of Candida spp. infections.

### Background of the invention

Global prevalence of health-care (acquired) infection (HCAI), also called nosocomial diseases is estimated between 7.1%- 15.5%, with an average mortality of 19-30% (Allegranzi et al., 2011, Lancet 2011; 377: 228-41)*.* Those nosocomial infection are caused by bacteria (70-75%), yeast/fungi (up to 22%) and virus (<10%) (Weiner et al., 2016, Infection Control & Hospital Epidemiology, 37(11*)*)*. Candida spp.* is the most common fungal pathogen (Pfaller et al. 2006, Current Epidemiological Trends; Clinical Infectious Diseases, 43:S3-14). In hospital patients, colonization by *Candida spp.* occurs in up to 80% of the patients (Eggimann et al., 2011, Annals of Intensive Care, 1:37*,* Eggimann et al., 2014, Intensive Care Med, 40:1429-1448*;* Quindos et al., 2014, Rev Iberoam Micol.;31(1):42-48*).*

Candidemia (systemic blood stream infections by *Candida spp.*) is the most faced consequence (75%) of invasive candidiasis. *Candida spp.* accounts for 8-22% of all health-care -acquired bloodstream infections (BSI) and the incidence of candidemia is usually tenfold higher in intensive care units than in healthy population (Pappas et al., 2018, Nature Review-Disease Primers, 18026(4*);* Calandra et al., 2016, Critical Care, 20:125*; Eggimann et al. 2011, supra).* The overall mortality attributable to candidemia is ranged from 10-47% according to the different types of studies but in some cases, mortality can be up to 70% (*Eggimann et al., 2011, supra; Pappas et al., 2018, supra*)*.*

As opposed to bacterial infections, invasive candidiasis is characterized by a 7-10-days delay between exposure to risk factors (colonization) and systemic infection development (candidemia), thus this delay is essential for an early diagnostic (*Eggimann et al., 2014, supra*)*. Candida spp.* is detected on the mucosal surface of 50-70% of healthy humans. When breaches in the intestinal barriers occur, *Candida spp.* can disseminate to the abdominal cavity directly and invade the bloodstream (candidemia). Under normal conditions, *Candida spp.* behaves as a commensal organism without causing disease. However, impairment of immune response (antibiotic treatment, HIV, immunodeficiency...) can promote fungal overgrowth in the gut and candidemia, which can lead to deep-seated opportunistic infections in various organs (invasive candidiasis) (*Pappas et al., 2018, supra*)*.* Invasion of the mucosa by *Candida albicans* is monitored by the morphological changes (yeast to hypha) of cell wall (Gow et al., 2012, Nature Review-Microbiology, 10 112-122)*.* During the yeast-to-hypha transition, genes encoding for hypha-specific proteins or *Candida albicans* germ tube protein (CAGT) such as hyphal wall protein 1 (Hwp1), hyphal regulated protein (Hyr1) and agglutinin-like sequence 3 (Als3) are upregulated (*Gow et al., 2012, supra*)*.*

To date, blood culture remains the gold standard for diagnosis of candidemia although a poor sensitivity 21-71% (*Pappas et al., 2018, supra;* Calandra et al., 2016, Critical Care, 20:125)*.* Furthermore, it lasts 2-4 days before *Candida albicans* is identified and proper therapeutic decisions can be made (Safavieh et al. 2017, Crit Rev Biotechnol., 37(4):441-458)*.* Moreover, blood cultures are rarely positive in patients with deep-seated invasive candidiasis (pre-candidemia stage) and lead to undiagnosed patients and inadequate overuse of wide spectrum antibiotics (*Calandra et al., 2016, supra;* Clancy et al. 2013, Clinical Infectious Diseases, 56(9):1284-92)*.*

To avoid labour intensive and uncertain blood culture diagnostic methods, several biomarkers have been targeted in reason of their high concentration in the blood stream like cell wall components of *Candida spp.* such as Mannan and 1,3-β-D-glucan (BDG). These assays are based on colorimetric or turbidimetric methods. The detection of BDG is the most used in microbiological laboratories and is FDA-approved but is labour intensive, time consuming and requires highly skilled people. The low specificity of BDG and Mannan testing is due to the fact that these markers not only identify Candida species infections but other fungal infections (Angebault et al., 2016, Open Forum Infectious Diseases, 3(3), 128*; Calandra et al., 2016, supra).* The sensitivity of the test is considered as 76-95% but the specificity widely varies between 40-92% (*Pappas et al., 2018, supra; Angebault et al., 2016, supra;* Lamoth et al., 2012, Clinical Infectious Diseases, 54(5):633-43*;* Posch et al., 2017, Expert Review of Anti-infective Therapy, 15(9):829-838*;* Leon et al., 2016, Critical Care, 20:149*).*

Well-known immunogenic Candida proteins have been intensively studied as biomarker of invasive candidiasis. *Candida albicans* germ tube family (CAGT) of proteins has been targeted to develop antibodies for the diagnosis of Candidiasis (Garcia-Ruiz et al., 1997, Journal of Clinical Microbiology, 35(12*)*)*.* More than 20 varieties of germ-tube specific antigens are known such as Hyphal wall protein 1 (Hwp1), Als3p, Ece1p, Hyr1p (Bikandi et al., 1998, Clinical and Diagnostic Laboratory Immunology, 5(3), 369-374 *;* Lain et al., 2008, Clinical and Developmental Immunology, Volume 2008, Article ID 721950, 7 pages*).* CAGT antigens are only present on the surface of the hyphal form of Candida species, being an interesting tool for the diagnostic of hyphal-mediated invasive candidiasis (*Bikandi et al., 1998, supra*)*.* Hwp1 serves as a substrate for mammalian transglutaminase, covalently linking *C. albicans'* hypha to the host cells (Mayer et al., 2013, Virulence, 4:2, 119-128*;* Staab et al. 1999, Science, 283*;* Nobile et al., 2006, Eukaryotic cell, 5(10) 1604 1610*).*

Various proteins located on the cell surface of *Candida albicans* have been identified as also potential diagnostic markers. The most studied and well-documented Candida cell surface proteins is enolase (Eno) but other proteins such as fructose-bisphosphate aldolase (Fba1), phosphoglycerate kinase (Pgk1) or β-glucosidase (Bgl2) were described as potential biomarkers (Lain et al., 2007b, Clinical and Vaccine Immunology, 14(3) 318-319*;* He et al., 2015, Front. Microbiol., 6:920*;* Li et al. 2013, BMC Infectious Diseases, 13:253*; Clancy et al. 2013, supra).* Lysine histone methyltransferase (Set1) and Protein similar to ferric reductase Fre10p (Cfl91) contribute to virulence and have been described as biomarkers for acute and convalescent candidemia patients with high antibody responses (Clancy et al., 2008, Journal of clinical Microbiology, 46(5) 1647-1654*;* Mochon et al., 2010, PLoS Pathog, 6(3*)*)*.*

Diagnosis of invasive candidiasis using ELISA with Hwp1 (UniProtKB - P46593) or its N-terminal fragments (amino acids 41-200 from Hwp1 full length) has recently been reported with good sensitivity (88.9%) and specificity (90.2%) (Lain et al. 2007a, BMC Microbiology, 7:35*;* Corouge et al., 2015, PLoS ONE, 10(3*);* Mochon et al., 2010, PLoS Pathog, 6(3*);* Naglik et al., 2006, Journal of Medical Microbiology, 55 1323-1327)*.* Positive response to anti-Hwp1 was detected 5 to 6 days before the isolation of C. *albicans* in blood (*Lain et al. 2007a, supra*)*.* WO 2014/001574 relates to methods for diagnosing invasive Candida infections based on the combined detection of "Candida glycan" (comprising Mannan, Glycan or Chitin) with known methods such as Fungitell^{®} (Associates of Cape Cod Inc.) or Platelia^{™} Candida antigen Plus test (Bio-Rad Laboratories) and the presence of antibody against of a Candia protein. Among the various possible antibodies against Candida proteins to be detected in combination with Candida glycan are mentioned anti-Hwp1 antibodies and in particular for this purpose, the use in an ELISA assay of N-terminal Hwp1 polypeptides as defined in *Lain et al., 2017, supra* (fragment 41-200 of Hwp1) or a fragment 40-187 as mentioned in Fradin et al., 2008, Infection and Immunity, 4509-4517*.* WO 2014/001574 describes an improved sensitivity of a combined detection of mannan antigens (monoclonal antibody EBCA1 of Platelia^{™} Candida antigen Plus test) and anti-Candida protein antibodies compared to the combined detection of mannan antigens and anti-mannan antibodies. Consequently, there is a very high need to develop accurate methods and tools for early stage detection of candidemia and invasive candidiasis, i.e. before the fungal infection reaches the systemic stage in order to allow applying the adequate treatment and reduce mortality and propagation of the disease to other patients.

### Summary of the invention

The present invention relates to the unexpected finding that a lateral flow assay based on a fragment of Hwp1 (Hwp1.3) allows achieving a sensitivity higher than known assays such as those based on Mannan detection (Chumpitazi et al., 2014, Medical Mycology, 52, 460 469*;* Mikulska et al., 2010, Critical Care, 14:R222*;* Held et al., 2013, Journal of Clinical Microbiology, 51(4), 1158-1164)*,* anti-mannan detection (*Held et al., 2013, supra; Mikulska et al., 2010, supra*) or gold-standard microbial blood analysis (*Pappas et al., 2018, supra; Calandra et al., 2016, supra; Clancy et al., 2013, supra*) and a quick response. The present invention further relates to the unexpected finding that the combination of the fragment of Hwp1 (Hwp1.3) with a cell surface protein allows the detection of both forms of the Candida infection, namely hyphal-mediated infection (invasive candidiasis) and the infection caused by non-hyphal Candida. In fact, mannan or beta-glucan are polysaccharides present on all type of cell surface of *Candida spp.* (*Gow et al., 2012, supra*) and detection of these biomarkers do not distinguish between hyphal and non-hyphal *Candida spp.* Blood culture analysis detect only the viable *Candida spp.* without distinction of the form. However, identification of the form of *Candida spp.,* responsible of the infection, can help in the management of treatment and prevention (e.g. catheter related infection, deep-seated invasive candidiasis...) and therefore the usefulness of a method of the invention are really promising.

An object of this invention is to provide a method for sensitive and specific detection of *Candida spp.* infection in a sample.

It is advantageous to provide a method for sensitive (e.g. >90%) and specific (e.g. >90%) detection of *Candida spp.* infection in whole blood, serum, plasma or bronchoalveolar lavage (BAL) samples before systemic infection stage (pre-candidemia stage).

It is advantageous to provide a method for sensitive and specific detection of *Candida spp.* infection that would allow discriminating *Candida spp.* infected samples from other fungal, bacterial or viral infections.

It is advantageous to provide a method for sensitive and specific detection of *Candida spp.* infection in bronchoalveolar lavage (BAL) samples to detect candida pneumoniae, for example for patients from intensive care unit and since BAL samples can be used for the detection of pneumoniae caused by *Candida albicans* the methods of the invention is quicker than actual microbiological analysis (Schnabel et al, 2014, Open Forum Infect Dis., 2014, 1(1*)*).

It is advantageous to provide a method for sensitive and specific detection of *Candida spp.* infection within few minutes.

It is advantageous to provide a method for sensitive and specific detection of *Candida spp.* infection, with a reduced rate of false negative (e.g. <5%).

It is advantageous to provide a method for sensitive and specific detection of invasive candidiasis (hyphal form of *Candida spp.*) and non-hyphal Candida infection.

Another object of this invention is to provide a test kit for sensitive and specific detection of *Candida spp.* infections in samples.

Objects of this invention have been achieved by providing a lateral flow immunoassay according to claim 1 or method according to claim 10.

Disclosed herein, according to a first aspect of the invention, is a lateral flow immunoassay device for qualitative or quantitative detection of Candida infection in a sample selected from a whole blood, serum, plasma and bronchoalveolar lavage (BAL) comprising a backing support and, on said backing support, a capillary flow array and a wicking pad. The capillary flow array comprises **i)** a sample receiving pad located at one end of the backing support; **ii)** a conjugate release pad being distinct from the sample receiving pad or included to the sample receiving pad and being in capillary contact with the sample receiving pad and being impregnated with at least one detection reagent comprising a first binder specific and binding to the anti-Hwp1 antibody analyte conjugated to a first label moiety; **iii)** a detection pad being distinct from the conjugate release pad and being in capillary contact with the conjugate release pad and comprising a detection membrane, a capture test reagent array comprising at least one reagent test line and a capture control reagent array comprising at least one control reagent line, said capture reagent and control reagent arrays being immobilized on the detection membrane, wherein the wicking pad is located at the other end of the backing support and being in capillary contact with the detection pad and wherein said at least one reagent test line comprises a Hwp1.3 protein, wherein a Hwpl.3 protein consists of SEQ ID NO: 1, and said at least one control reagent line comprises a control reagent having a specific affinity for the first binder of the detection reagent. The said at least one detection reagent are diffusively releasable from the conjugate release pad. The conjugate release pad comprising a detection reagent support wherein the said at least said one detection reagent is bound in a capillary releasable manner, receives the sample (e.g. blood, serum, plasma or bronchoalveolar lavage (BAL)) from the sample receiving pad and releases the detection reagent from its detection reagent support and a first immuno-complex is formed when the anti-Hwp1 antibody analyte is present in the sample and is combined with the detection reagent. The first immuno-complex migrates to the detection pad where it binds to the said at least one reagent test line comprising the Hwp1.3 protein. The excess of detection reagent is captured by the said control reagent line.

Disclosed herein, according to another aspect of the invention, is a method for measuring the content of anti-Hwp1 antibodies in a sample, said method comprising:
a) Providing a previously obtained sample selected from a whole blood, plasma, serum and bronchoalveolar lavage (BAL) potentially contaminated with *Candida spp.*, diluted or not in aqueous solution;
b) Subjecting the said sample to a lateral flow immunoassay comprising **i)** a conjugate release pad comprising detection reagents for said antibodies and **ii)** a detection pad comprising a detection membrane where at least Hwp1.3 protein is bound;
c) Detecting the presence or absence of a detection line on the detection membrane at the location where the said least Hwp1.3 protein is bound, the presence of said detection line being indicative of the presence of anti-Hwp1 antibodies, wherein a Hwpl.3 protein consists of SEQ ID NO: 1.

According to another aspect of the invention, is provided a kit for qualitative or quantitative detection of Candida infection in a sample, said kit comprising at least one lateral flow immunoassay device according to the invention.

### Description of the figures

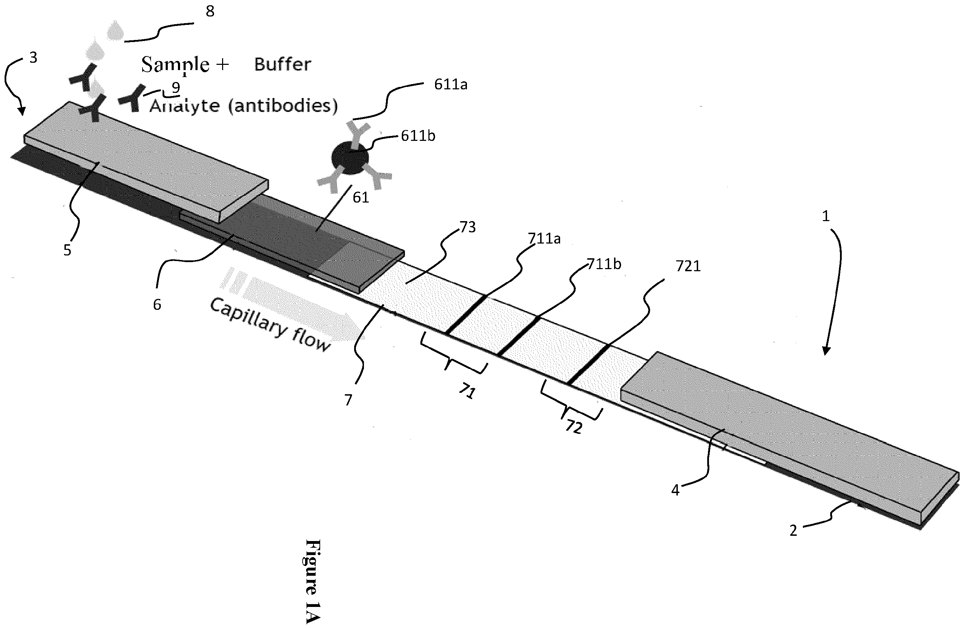

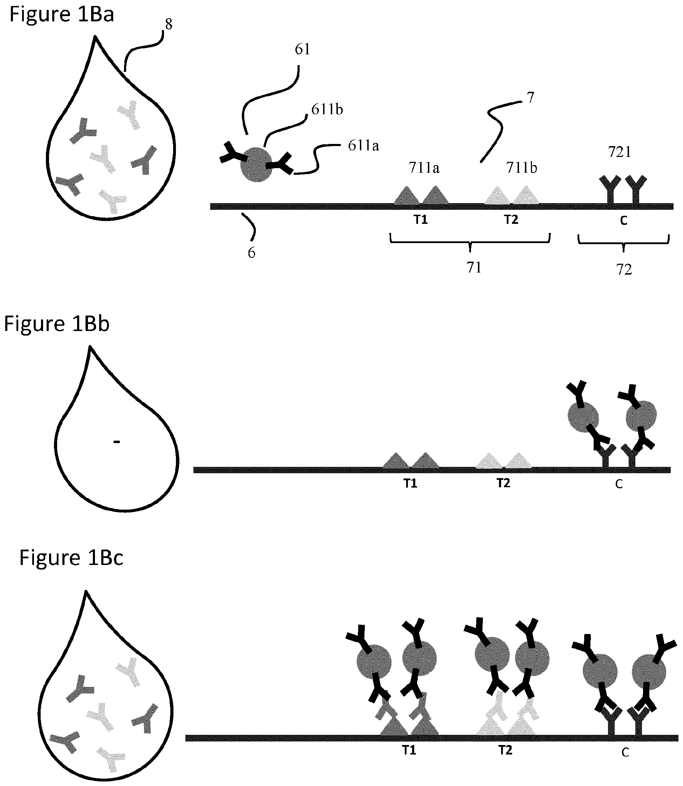

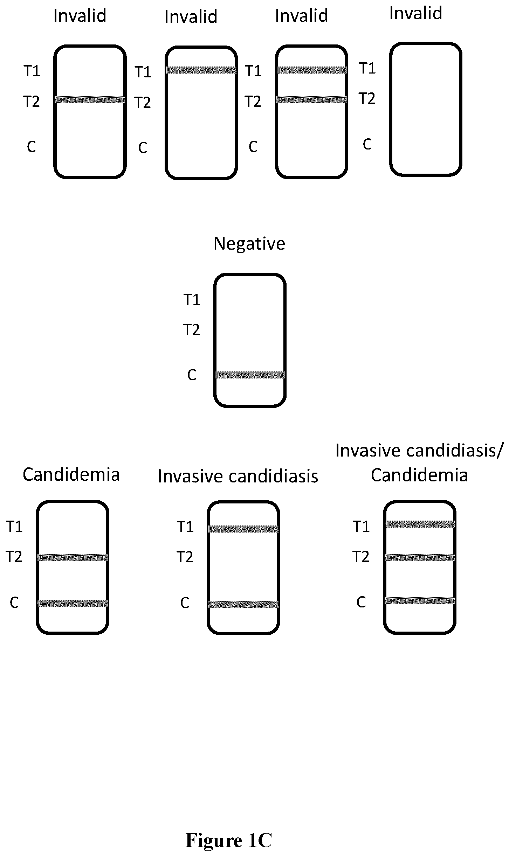

**Figure 1** is a schematic representation of a lateral flow immunoassay of the invention (**A**), the main elements used in the method (**B**) of the invention and its read outs (**C**). **B:** In a method of the invention, in particular using a lateral flow immunoassay of the invention such as described under **A**, the sample (8) containing the analyte (Human anti-Hwp1 and/or anti-Candida protein antibodies) is subjected to the sample receiving pad and migrates to the conjugate release pad comprising detection reagents for said antibodies (first binder with first label moiety) which are then released and migrate with the sample to the detection pad comprising a detection membrane where at least Hwp1.3 protein is bound (T1: test line 1) and optionally at least one further candida protein (T2: test line 2) and a control agent specific to first binder (C: control line). In case no analyte is present (test negative), the detection reagents will only bind the control line (**Figure 1Bb**) and in case of the analyte is present (test positive), the analyte (human anti-Hwp1.3 and/or anti-Candida protein antibodies) will bind to specific Hwp1.3 or Candida protein on the surface of the detection pad and detection reagent (e.g. mouse anti-human IgG antibodies with first label moiety) will be attached to the analytes (human IgG antibodies fixed to test lines proteins) on the detection pad and the control line (e.g. goat anti-Mouse antibody) captures excess of detection reagent **(****Figure 1Bc****); C:** The read outs are in the form of a test line **(T)** formed by the immune-complex formed by the analyte (e.g. anti-Hwp1 or anti-Candida cell surface protein antibody), the capture test reagent (e.g. Hwp1.3 or Candida cell surface protein) and the detection reagent(s) (e.g. Gold nanoparticle conjugated with mouse anti-human IgG) and the control line (**C**) is formed by the immune-complex formed by the capture control reagent (e.g. anti-mouse antibody like a Goat anti-mouse antibody) and the detection reagent(s) (e.g. Gold nanoparticle conjugated with mouse anti-human IgG). Several examples of read-out are provided as an example.

### Detailed description

The term "Hwp1.3 protein" refers to a protein consisting of SEQ ID NO: 1. The term "conservative variant", applied to a peptide or polypeptide, as referred to herein means a peptide or polypeptide substantially homologous to the referenced peptide sequence, but which has at least one amino acid different from that of the referenced sequence because of one or more amino acid deletion, insertion and/or substitution. Substantially homologous means a variant amino acid sequence which is identical to the referenced peptide sequence except for the deletion and/or substitution of 1, 2, 3, 4, 5 or 6 amino acid residues. In a more particular embodiment, a variant amino acid sequence is identical to the referenced peptide sequence except for the deletion and/or conservative substitution of 1, 2, 3, 4, 5 or 6 amino acid residues. The identity of two amino acid sequences can be determined by visual inspection and/or mathematical calculation, or more easily by comparing sequence information using known computer program used for sequence comparison such as Clustal package version 1.83. A variant may comprise a sequence having at least one conservatively substituted amino acid, meaning that a given amino acid residue is replaced by a residue having similar physicochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Amino acid hydrophobicity can be found on the basis of known scales such as Kyte, et al, 1982, J. Mol. Biol., 157: 105- 131*;* Eisenberg, 1984, Ann. Rev. Biochem., 53: 595-623*.* Other such conservative substitutions, for example, substitutions of entire regions having similar hydrophobicity characteristics or rigid coiled structure of Hwp1 or substitution variants with planar secondary structure, for example assessed by circular Dichroism or Infrared spectroscopy or using any bioinformatic tools to predict protein secondary structure and rigid helical wheel properties such as Advanced Protein Secondary Structure Prediction Server (APSSP) or any other tools which are well known to the skilled persons (Kyte, et al, 1982, supra). For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired. Exemplary amino acid substitutions are presented in Table 1 below. The term "variant" also includes a peptide or polypeptide substantially homologous to the referenced peptide sequence, but which has an amino acid sequence different from that of the referenced sequence because one or more amino acids have been chemically modified or substituted by amino acids analogs. For example, non-natural residues can be introduced to enhance the immuno-reactivity with antibodies or binding properties (Geurink et al., 2013, J. Med. Chem., 56, 1262*;* Rand et al., 2012, Med. Chem. Commun, 3, 1282*).*

**Table 1**

| **Amino acids** | **Examples of « conservative » substitutions** |
|---|---|
| Ala (A) | Val, Leu, Ile |
| Arg (R) | Lys, Gln, Asn |
| Asn (N) | Gln |
| Asp (D) | Glu |
| Cys (C) | Ser, Ala |
| Gln (Q) | Asn |
| Glu (E) | Asp |
| Gly (G) | Pro, Ala |
| His (H) | Asn, Gln, Lys, Arg |
| Ile (I) | Leu, Val, Met, Ala, Phe, Norleucine |
| Leu (L) | Ile, Val, Met, Ala, Phe, Norleucine |
| Lys (K) | Arg, Gln, Asn |
| Met (M) | Leu, Ile, Phe |
| Phe (F) | Leu, Val, Ile, Ala, Tyr |
| Pro (P) | Ala, Gly |
| Ser (S) | Thr, Ala, Cys |
| Trp (W) | Phe, Tyr |
| Thr (T) | Ser |
| Tyr (Y) | Trp, Phe, Thr, Ser |
| Val (V) | Ile Met, Leu, Phe, Ala, Norleucine |

According to a particular embodiment, C-term fragments of a protein used in the invention have about 13 to about 25 amino acids, such as for example 14 to about 20 amino acids. For example, a Hwp1.3 fragment is a peptide having a sequence QEPCDYPQQPQEPCDYPQQP (**SEQ ID NO: 10**) as described in *Corouge et al., 2015,* supra.

According to another particular embodiment, the peptides can be optionally acetylated at the N-terminus and/or amidated at the C-terminus or could be conjugated to a carrier, such as a carrier protein, for example Bovine Serum Albumin BSA or other small molecules for conjugation/binding to the surface of the detection membrane or to increase the sensibility. For example, according to a particular embodiment, when the detection membrane is a nitrocellulose membrane, peptide fragments (13 to 25 amino acids) are attached to the detection membrane through a carrier such as a carrier protein. The term "Eno protein" refers to enolase (Eno) protein (UniProtKB - P30575) as described in *Li et al. 2013, supra* and any conservative variants thereof. The term "Eno protein" further covers a fragment of a protein of **SEQ ID NO: 4** (Eno fragments). According to a particular embodiment, C-term fragments have about 13 to about 25 amino acids, such as for example 14 to about 20 amino acids. For example, Eno fragment is a peptide having a sequence DSRGNPTVEVDFTT (**SEQ ID NO: 11**) as described in Xin et al., 2008, PNAS 105(36), 13526-13531*.*

The term "Bgl2 protein" refers to β-glucosidase (Bgl2) protein (UniProtKB - Q5AMT2) as described in *He et al. 2015, supra* and any conservative variants thereof. The term "Bgl2 protein" further covers a fragment of a protein of **SEQ ID NO: 5** (Bgl2 fragments). According to a particular embodiment, C-term fragments have about 13 to about 25 amino acids, such as for example 14 to about 20 amino acids.

The term "Fba1 protein" refers to fructose-bisphosphate aldolase (Fba1) (UniProtKB - Q9URB4) as described in *Li et al. 2013, supra* and any conservative variants thereof. The term "Fba1 protein" further covers a C-term fragment of a protein of **SEQ ID NO: 6** (Fba1 fragments). According to a particular embodiment, fragments have about 13 to about 25 amino acids, such as for example 14 to about 20 amino acids. For example, Fba1 fragment is a peptide having a sequence YGKDVKDLFDYAQE (**SEQ ID NO: 12**) as described in *Xin et al., 2008, supra* and in Shi et al., 2018, Vaccine, 36(38): 5717-5724.

The term "Pgk1 protein" refers to phosphoglycerate kinase (Pgk1) protein (UniProtKB - P46273) (sequence of amino acid 1-238 described in Clancy et al., 2008, Journal of clinical Microbiology 2008; 46(5) 1647-1654*)* and any conservative variants thereof. The term "Pgkl protein" further covers a fragment of a protein of **SEQ ID NO: 7** (Pgk1 fragments). According to a particular embodiment, C-term fragments have about 13 to about 25 amino acids, such as for example 14 to about 20 amino acids. For example, Pgk1fragment is a peptide having a sequence VPLDGKTITNNQRI (**SEQ ID NO: 13**) as described in *Xin et al., 2009, supra.*

The term "Set1 protein" refers to Lysine histone methyltransferase (Set1) protein (UniProtKB - Q5ABG1) (**SEQ ID NO: 8**) as described in *Clancy et al., 2008, supra* and any conservative variants thereof. Set1 was previously shown to contribute to virulence and to have higher antibody responses among patients with systemic candidiasis than among controls. *Clancy et al., 2018, supra* have shown that Set1 protein represent one of the best biomarkers for the diagnostic of systemic candidiasis with sensitivity of 98.3%.

The term "Cfl91 protein" refers to Protein similar to ferric reductase Fre10p (Candida Genome Database - CR_06870C_A) (**SEQ ID NO: 9**) as described in *Mochon et al., 2010, supra* and any conservative variants thereof. Cfl91 is a putative ferric reductase similar to Fre10, which is required for the release of iron from transferrin and the reduction to ferrous iron. Cfl91 is found as a biomarker for both acute and convalescent candidemia patients (*Mochon et al., 2010, supra*)*.*

The term "whole blood sample" is intended to mean any blood sample diluted or not and/or placed in aqueous solution.

The term "aqueous solution" is intended to mean containing water or placed in water e.g. purified, distilled, sterile water or water for injection.

The expression "container" refers to any recipient suitable for sample collection, sample dilution and sample preparation such as vials, centrifuge tubes, flasks, Eppendorf tubes, micro-centrifuge tubes, U-shaped tubes, blood collection tubes, thistle tubes, hybridization tubes, capillary tubes, wintrobe tubes, culture tubes, micro-titer tubes, hematocrit tubes and micro-hematocrit tubes.

### Assay of the invention

Referring to the figures, in particular first to Figure 1A, a lateral flow immunoassay device for qualitative or quantitative detection of Candida infection in a sample selected from a whole blood, plasma, serum and bronchoalveolar lavage (BAL) comprises:
- a backing support 2
- a capillary flow array 3 on said backing support,
- a wicking pad 4,
the capillary flow array 3 comprises **i)** a sample receiving pad 5 located at one end of the backing support; **ii)** a conjugate release pad 6 being distinct from the sample receiving pad or being integrated to the sample receiving pad and being in capillary contact with the sample receiving pad 5 and being impregnated with at least one detection reagent 6 comprising a first binder specific and binding to the Human IgG antibodies analyte 611a conjugated to a first label moiety 611b; **iii)** a detection pad 7 being distinct from the conjugate release pad and being in capillary contact with the conjugate release pad and comprising a detection membrane 73, a capture test reagent array 71 comprising at least one reagent test line 711a and a capture control reagent array 72 comprising one control reagent line 721, said capture reagent and control reagent arrays being immobilized on the detection membrane 73, the wicking pad 4 being at the other end of the backing support 2 and being in capillary contact with the detection pad 7, wherein said at least one reagent test line 711a comprises a Hwp1.3 protein and said control reagent line comprises a control reagent having a specific affinity for the first binder 61. Optionally, the capture test reagent array 71 may comprise further reagent test lines 711b,...n, such Eno, Fba1 etc... proteins.

The conjugate release pad 6 comprises a detection reagent support 62 wherein the said at least said one detection reagent 61 is bound in a capillary releasable manner, receives sample 8 (e.g. whole blood, plasma, serum or BAL) from the sample receiving pad 5 and releases the detection reagent 61 from its detection reagent support 62 and a first immuno-complex is formed when an antibody analyte 9 (e.g. human anti-Hwp1 IgG or other specific antibodies such as human anti-Eno, human anti-Fba1, IgG antibodies) is present in the sample and is combined with the detection reagent 61. The first immuno-complex migrates to the detection pad 7 where it binds to the said at least one reagent test line 711a or 711b, 711n (depending of the antibody present in the sample) from the capture reagent array 71 comprising the Hwp1.3 protein. The excess of detection reagent 61 is captured by the said one control reagent line 721a from the capture control reagent array 72.

According to a particular embodiment, the backing support is made of a non-porous material such as polyvinyl chloride (PVC).

According to another particular embodiment, the sample receiving pad has pores so as to receive the whole blood, plasma, serum or BAL sample. Typically, the sample receiving pad comprises an absorbant pad on to which the sample is applied and is typically composed of a woven mesh or cellulose filter. Irrespective of which material is chosen, the sample receiving pad should exhibit consistent absorbency, thickness and density so that uniform wicking rates ensure assay reproducibility. The sample receiving pad should also have low protein binding to avoid loss of analyte. Pre-treatment such as blocking with protein (e.g. BSA, milk protein, gelatine, ...) or polymer/surfactant could be applied to the sample receiving pad to reduce non-specific binding of the analyte. The sample receiving pad could be designed in such way to have red blood cells retention properties.

According to another particular embodiment, the conjugate pad could be made of the same material/support than the sample receiving pad.

According to another particular embodiment, the detection reagent support of the conjugate release pad is typically composed of non-woven glass fiber into which the detection reagent has dried. Once the sample receiving pad has been saturated, the sample flows in to the conjugate release pad where it releases the detection reagent(s) which in turn leave(s) the conjugate release pad and moves with the sample in the detection reagent support to the detection membrane of the detection pad.

According to another particular embodiment, the detection reagent is specific to human IgG (e.g. to human anti-Hwp1.3 IgG, human anti-Eno IgG, human anti-Fba1 IgG etc), such as an anti-human IgG antibody like a mouse or rabbit anti-human IgG antibody (first binder specific and binding to the antibody analyte) conjugated to colored or fluorescent particles (label moiety).

According to another particular embodiment, the detection reagent is specific to human IgA.

According to a particular aspect, the label moieties can be gold nanoparticles, latex beads, cellulose beads, fluorescent labels/particles and other colloidal metals and magnetic particles which produce a colored/fluorescent read out or any other particles generally used in lateral flow assays. According to a further embodiment, the label moiety is a gold, cellulose or latex particle.

According to a particular aspect, the detection membrane can be made a membrane of nitrocellulose membrane or cellulose acetate, polyvinylidene fluoride (PVDF), charge-modified nylon, polyethersulfone (PES) or treated/non-treated cellulose. Typically, nitrocellulose membranes exhibit a range of pore sizes (0.05 to 12 µm) and are suitable for the present assay.

According to a particular aspect, capillary flow values expressed in Capillary speed down web for purified water (s/40mm) between 90 and 180 are suitable for the present assay.

Capture reagents are immobilized across the detection membrane, typically in two capture reagent arrays (test and control reagents), respectively and each of the capture reagent arrays comprise one or more lines. The test line(s) is used to bind the sample target analyte (e.g. antibodies, while the control line consists of agents specific for the detection of the first binders such as species-specific antibodies (e.g. anti-mouse/anti-rabbit antibodies) and is used to demonstrate that the lateral flow immunoassay is performing as it should be.

According to a particular aspect, the wicking pad is composed of a cellulose/cotton fiber.

According to a particular aspect, the lateral flow immunoassay may be inserted into a rigid housing (e.g. a plastic cassette) protecting the capillary flow array and wicking pad and having an opening over the sample receiving pad for loading the sample on to said sample receiving pad and one opening over the capture reagent array for the visual interpretation of the test (presence/absence of colored lines).

According to a particular embodiment, the detection pad may comprise more than one reagent test line in the capture test reagent array. In particular, the capture test reagent array may comprise a Hwp1.3 protein and at least one Candida cell surface protein, such as for example selected from Eno, Bgl2, Fba1, Pgk1, Set1 and Cfl91.

According to a particular embodiment, the control agent specific to first binder is a species-specific antibody to the anti-human IgG or IgA antibody bound to detection particles (e.g. anti-mouse or anti-rabbit).

### Preparation of an immunoassay of the invention

A sample receiving pad can be purchased from Ahlstrom-Munksjö CytoSep^{®} as a single layer plasma separation media consisting of high purity natural and synthetic fibers for whole blood diagnosis such as Ref. A1660, A1667 or A1668. Pre-treated or not conjugate pads can be made of glass and/or polyester fibers such as Ahlstrom-Munksjö Ref. A8964 or ReliaFlow^{™}. According to a particular embodiment, the detection membrane can be made of nitrocellulose such as Hi-Flow^{™} Plus from Millipore, HF-120, HF-135 or HF-180 (preferred), representing the capillary flow time (sec/4cm). Treatment of the detection membrane with blocking proteins such as BSA, casein, gelatin, milk protein or polymers/surfactant could be realized to reduce background signal and to reduce non-specific binding of analyte. The wicking pad serves as absorbent of liquid excess and needs to have good absorbent properties. For example, cotton fiber from Ahlstrom-Munksjö Ref. A222 or A 237 can be used.

Recombinant proteins can be used as capture reagent test proteins (Hwp1.3, Eno...) for the test lines, i.e. produced in *Escherichia coli* and diluted to a concentration between 0.1 and 1 mg/ml in as PBS or other low salt buffer. Addition of blocking/carrier proteins, surfactant or alcohol (ethanol) could be used to optimize the binding and drying of test lines onto the detection membrane. Polyclonal antibodies (e.g. Goat anti-mouse antibody) with minimal cross-reaction to serum protein and other species, are diluted in PBS or other low salt buffer to a concentration between 0.1 and 1 mg/ml can be used as control agent.

Monoclonal mouse anti-human IgG antibody conjugated to detection particles can be sued as detection reagents.

All paper and membrane (sample receiving pad, conjugate pad, detection membrane and wicking pad) could be pre-treated and are cut with respective width, depending of the length of the capillary flow array and the superposition of the different membranes. If pre-treatments with blocking solution occur, treated membrane need to be dried in specific condition (such as 37°C, 10% RH (relative humidity), 2 hours or overnight at room temperature and 10% RH). Spraying of the detection reagent on the detection reagent support from the conjugate release pad can be realized with a dispensing machine (BioDot or Kinematic Automation) with air pressure (e.g. 2 PSI), pump dispense flow of e.g. 1 *µ* l/cm and bed speed of e.g. 5 cm/second.

Sprayed detection reagent support can be dried with same condition as above. Recombinant proteins (test lines from capture test reagent array) and capture control antibody (control line) are diluted and applied in different position on the detection membrane using similar dispensing machine, (e.g. with pump dispense flow of 1 *µ* l/cm and bed speed of 5 cm/second). Detection membrane is dried under same condition cited above. Assembling of all components of the assay is made by hand or with an automatized process. Assembled cards with different paper and membrane could be covered with a cover tape (transparent adhesive plastic) in order to protect the assay integrity. Assembled cards are then cut in strip suitable for the assay (e.g. with a width between 3.0 and 7.0 mm). Cut strips can then be assembled in a plastic cassette in order to protect the strip, pouched with a desiccant in an aluminum pouch and sealed with a thermo-sealing machine. All production and assembling steps take place in clean room with controlled temperature (<25°C) and humidity (<40%RH). Assembling of the final kit with accessories (optional), test in sealed pouch, instruction for use in a kit box can be done in a non-controlled environment.

### Methods of the invention

Disclosed herein, according to a second aspect of the invention, is a method for measuring the content of anti-Hwp1 antibodies in a sample, said method comprising:
a) Providing a previously obtained sample selected from a whole blood, serum, plasma and bronchoalveolar lavage (BAL) potentially contaminated with Candida spp., optionally diluted in aqueous solution;
b) Subjecting the said sample to a lateral flow immunoassay comprising **i)** a conjugate release pad comprising detection reagents for said antibodies and **ii)** a detection pad comprising a detection membrane where at least Hwp1.3 protein is bound;
c) Detecting the presence or absence of a detection line on the detection membrane at the location where the said least Hwp1.3 protein is bound, the presence of said detection line being indicative of the presence of anti-Hwp1 antibodies, wherein a Hwpl.3 protein consists of SEQ ID NO: 1.

According to a particular aspect, the detection of the presence of anti-Hwp1 antibodies according to a method of this invention or by an immunoassay of the invention is indicative of hyphal-mediated Candida infection, i.e. invasive candidiasis.

According to another further particular aspect, is provided a method according to the invention wherein at least one Candida cell surface protein, for example selected from Eno, Bgl2, Fba1, Pgk1, Set1 and Cfl91 or a combination of those, is further bound to the detection membrane of said lateral flow immunoassay and the detection of the presence of antibodies to this said at least one Candida cell surface protein is further carried out under step c). The detection of the presence of anti-Candida cell surface protein antibodies according to a method of this invention or by an immunoassay of the invention is indicative of non-hyphal-mediated Candida infection.

According to another further particular aspect, is provided a method according to the invention wherein at least one Candida cell surface protein is Eno.

According to another further particular aspect, is provided a method according to the invention wherein at least one Candida cell surface protein is Bgl2.

According to another further particular aspect, is provided a method according to the invention wherein at least one Candida cell surface protein is Fba1.

According to another further particular aspect, is provided a method according to the invention wherein at least one Candida cell surface protein is Pgk1.

According to another further particular aspect, is provided a method according to the invention wherein at least one Candida cell surface protein is Set1.

According to another further particular aspect, is provided a method according to the invention wherein at least one Candida cell surface protein is Cfl91.

### Kits of the invention

According to a particular aspect of the invention, the said at least one lateral flow immunoassay device is sealed in a pouch with a desiccant.

According to a particular aspect of the invention, the said at least one lateral flow immunoassay device is assembled in a transparent plastic cassette.

According to a particular aspect of the invention is provided a kit of the invention may further comprise at least one of the following:
- a sample collection device (e.g. pipette or automatic lancet for finger prick blood sample or capillary tube, capillary pipette);
- a container with a buffering solution for dilution of the sample or for running the test with pure sample (e.g. Low-density polyethylene (LDPE) plastic bottle with cap and dropper or any other closed plastic bottle with dropper);
- instructions for use.

According to a particular aspect of the invention is provided a kit of the invention may optionally further comprise or more of the following:
- a disinfection pad;
- an adhesive plaster;
- a data reader for reading the assay results and optionally transmitting to a database (e.g. patient hospitalization data set).

According to a further aspect, is provided a kit according to the invention for conducting a method of the invention and its use in a method according to the invention.

According to a further aspect, an assay or a kit according to the invention are useful for detection of hyphal-mediated infection (invasive candidiasis).

According to a further aspect, an assay or a kit according to the invention are useful for detection of both forms of the Candida infection, namely hyphal-mediated infection (invasive candidiasis) and Candida infection caused by non-hyphal Candida.

According to a particular embodiment, the method and assay according to the invention allow the detection of a Candida infection in a very rapid manner (less than 30 minutes) at a very early stage of the infection (i.e. before the systemic bloodstream infection stage) with a high selectivity and high sensitivity (e.g. >90%).

Examples illustrating the invention will be described hereinafter in a more detailed manner and by reference to the embodiments represented in the Figures.

### EXAMPLES

The following abbreviations refer respectively to the definitions below:
**BSA** (bovine serum albumin); **PBS** (Phosphate-Buffered Saline); **TBS** (Tris-Buffered Saline) **TBST** (Tris-Buffered Saline with Tween).

### Example 1: Preparation of a lateral flow assay of the invention and comparison of performance

A lateral flow immunoassay of the invention was prepared as follows:

### Production of the "half-sticks " containing only the detection and wicking pads on the backing support

The production of half-sticks for the liquid phase of the test has been performed in a clean, controlled room (EU GMP Class D), temperature (<25°C) and humidity (<40% RH). A nitrocellulose membrane (width 25 mm) HF-180 (Merck Millipore) was used as detection membrane. Bands of absorbent pad A222 (Ahlstrom-Munksjö) with a width of 21 mm were cut and paste on the upper part of the backing support and used as wicking pad. Strips were cut with a width of 5 mm (± 0.5 mm) and stored in aluminum pouches with desiccants at room temperature.

### Spotting of biomarkers on the detection membrane of the half-sticks

Several biomarkers were used as test reagents: the biomarkers were each diluted at a specific concentration, indicated in Table 1 below, in TBS 1X. Then, 0.5µL of each diluted biomarker were spotted on the nitrocellulose membrane (detection membrane) and let dry at 25°C, <40%RH for 2 hours to form a test reagent line. Hwp1.3 corresponds to the test protein used in the invention which sequence (**SEQ ID NO: 1**) corresponds to amino acids 40-187 of full length Hwp1. Other fragments of full length Hwp1, namely fragment 41-200, Hwp1.2 (**SEQ ID NO: 2**) used in the ELISA and immunoblotting assay of *Lain et al., 2017, supra* and fragment 27-203 Hwp1.1 (**SEQ ID NO: 3**) WO 2014/001574 and fragment 27-203 described in *Fradin et al., 2008, supra* were used as comparatives.

**Table 1**

| **Test reagents** | **Sequence** | **Supplier ref.** | **Concentration [mg/ml]** |
|---|---|---|---|
| Hwp1.3 | SEQ ID NO: 1 | Genscript | 0.16 |
| Hwp1.2 (comparative) | SEQ ID NO: 2 | Genscript | 0.11 |
| Hwp1.1 (comparative) | SEQ ID NO: 3 | Cusabio, CSB-YP343212CZD | 0.4 |
| Candida Eno | SEQ ID NO: 4 | Diarect, 45301 | 0.6 |
| Candida Bgl2 | SEQ ID NO: 5 | Diarect, 44801 | 0.5 |
| Candida Fba1 | SEQ ID NO: 6 | MyBioSource MBS1345647 | 1.0 |

### Test with clinical samples

Positive samples serum (n=6) of patients with Candida sp. in blood were purchased from Discovery Life Sciences (Huntsville, USA) or were selected in the sera-bank from Augurix SA (Monthey, Switzerland). These samples were preliminary selected on the basis of the presence of *Candida albicans* in blood or suspected infection with Candida sp. Negative samples (n=8) were selected from the sera-bank of Augurix SA. All samples (n=14) were sent to Unilabs Switzerland (Coppet, Switzerland) for characterization using mannan and anti-mannan test (ELISA Fungitell^{™} and Platelia ^{™}).

Each Samples (25 µL) were mixed with 6 µL of 40 nm-Gold nanoparticles anti-human IgG (NanoImmunoTech, Zaragoza, Spain) used as detection agents and 2 µL of Tween 20 (Sigma-Aldrich, Darmstadt, Germany) on 96-well plate. Detection membranes (cellulose half-sticks prepared as described above) were added onto each well and the solution migrated during 5 minutes on the strip. Strips were visually read and results were expressed as positive (presence of candidemia) or negative (absence of candidemia) depending on the color of the spot: If a red line appears, it means a positive test and no red line means a negative test (see Figure 1C). Threshold is fixed at Rann score 4 standardly used for lateral flow assays to define visually the intensity of a colored line (the scale goes from 0 (no line) to 10 (line with high intensity).

All sera were preliminary characterized using available data such as microbial analysis (C. *albicans*), disease state (celiac positive, lactose intolerance) and in order to confirm the first characterization, samples were sent to Unilabs for a re-characterization using mannan/anti-mannan tests based on ELISA (in order to have comparative analysis).

Based on these results, diagnostic performances of each biomarker were calculated separately, by excluding samples where characterization not available for a sample size of n=14. The sensibility, specificity, positive predictive value (PPV) and negative predictive value (NPV) were calculated as described in Parikh et al., 2008, Indian J Ophthalmol., 56(1): 45 50 and listed in Table 2 below.

**Table 2**

| **Test reagents** | **Sensibility %** | **Specificity %** | **PPV %** | **NPV %** |
|---|---|---|---|---|
| **Hwp1.3** | **83.3** | **75** | **71.4** | **85.7** |
| Candida Eno | 66.7 | 100 | 100 | 80 |
| Candida Bgl2 | 66.7 | 100 | 100 | 80 |
| Candida Fba1 | 66.7 | 100 | 100 | 80 |
| Hwp1.1 | 66.7 | 87.5 | 80 | 77.8 |
| Hwp1.2 | 33.3 | 100 | 100 | 66.7 |

Diagnostic performances are clearly above the average for the test proteins Eno, Bgl2, Fba1 and Hwp1.3. Combination of Hwp1.3 with Eno, Bgl2 or Fba1 give always diagnostic performances above the average. Comparative fragment Hwp1.2, used in *Lain et al, 2007, supra* shows low sensitivity. This fragment was reported to present a low sensitivity (27.8%) by immunoblotting and high sensitivity (88.9%) by ELISA.

The best sensitivity of the later flow assay was obtained with Hwp1.3 used as test protein and the assay has similar performances than the reported performances for ELISA described in *Lain et al., 2017, supra.*

### Control with specific antibodies

In order to control the specificity of the test, specific antibody anti-Hwp1 (mAb 16B1, Lot.051118, as described in Marot-Leblond et al., 2000, Journal of clinical Microbiology, 38(1), 61-67*)* (KaliDiv, SARL, Arvillé, France) was serial diluted (dilution following a logarithmic scale) in TBST + 1% BSA containing goat anti-mouse IgG conjugated to cellulose nanoparticles (Asahi Kasei, Japan).

Detection membranes containing Hwp1.3 or the control fragments Hwp1.1 or Hwp1.2 (nitrocellulose half-sticks prepared as described above) used as test reagent lines (concentration Table 1) were added onto each well and the solution migrated during 10 minutes on the strip. The limit of detection (LOD), representing the lowest dilution where the signal is still positive, was determined for each Hwp1 fragment.

The reactivity and specificity of all fragment of Hwp1 was confirmed with the specific anti-Hwp1 monoclonal antibody and the limit of detection was lower for Hwp1.2 (dilution 1/2000) compared to Hwp1.1 and 1.3 (1/5000). This lowest sensitivity of Hwp1.2 could explain the lowest diagnostic performances of this fragment.

The above data support that the use of Hwp1.3 as a test protein in an immunoassay of the invention, alone or in combination with some candida cell surface protein (combination lead to diagnostic performances above the average) show similar or better diagnostic performances (sensibility) than those obtained with actual commercial tests including Mannan/anti-mannan (43-65%) (Serion^{™} or Platelia ^{™}) or β-glucan assays (77-82%) (Fungitell^{™})) The use of Hwp1.3 as a test protein in a lateral flow immunoassay of the invention performs much better in terms of sensitivity than the fragment used in the ELISA assay described in WO 2014/001574 as having high sensitivity (88.9% decreased to 33.3% when used in a later flow).

Therefore, a lateral flow immunoassay of the invention has the main advantages of the rapidity of detection (10-15 minutes compared to 2-3 days for existing ELISA) and the predictivity, allowing the detection of the infection before the systemic bloodstream infection stage (e.g. 5-6 days before the detection of the pathogen in blood) which is very promising for the development of a commercial assay which responds to the current needs in the field for a point of care test allowing to get the right information to quickly start an appropriate treatment to reduce the mortality and further contamination risks.

### List of elements referenced in the figures

1 Lateral flow immunoassay
   2 backing support
   3 capillary flow array
      5 sample receiving pad
      6 conjugate release pad
         61 detection reagent
            611a first binder to antibody analyte
            611b first label moiety
         62 detection reagent support
      7 Detection pad
         71 capture test reagent array
            711a test protein (Hwp1.3)
            711b optional further test protein (e.g. Bgl2 or Fba1 or Pgk1 or Set1 or Cfl91
         72 capture control reagent array
            721a control agent specific to first binder (e.g. species-specific antibody for the detection reagent)
         73 detection membrane
   4 wicking pad

### SEQUENCE LISTING

**SEQ ID NO: 1 - Hwp1.3 (synthetic fragment 40-187 from *Candida albicans* Hwp1)** 
SYDYYQEPCDDYPQQQQQEPCDYPQQQQEEPCDYPQQPQEPCDYPQQPQEPCD
YPQQPQEPCDYPQQPQEPCDNPPQPDVPCDNPPQPDVPCDNPPQPDVPCDNPPQPDVP
CDNPPQPDQPDDNPPIPNIPTDWIPNIPTDWIPD.
**SEQ ID NO: 2- comparative Hwp1.2 (synthetic fragment 41-200 from *Candida albicans* Hwp1)** 
YDYYQEPCDDYPQQQQQEPCDYPQQQQEEPCDYPQQPQEPCDYPQQPQEPCDY
PQQPQEPCDYPQQPQEPCDNPPQPDVPCDNPPQPDVPCDNPPQPDVPCDNPPQPDVPC
DNPPQPDQPDDNPPIPNIPTDWIPNIPTDWIPDIPEKPTTPATTPN.
**SEQ ID NO: 3 - comparative Hwp1.1 (synthetic fragment 27-203 from *Candida albicans* Hwp1)** 
GQGETEEALIQKRSYDYYQEPCDDYPQQQQQEPCDYPQQQQEEPCDYPQQQPQE
PCDYPQQPQEPCDYPQQPQEPCDYPQQPQEPCDNPPQPDVPCDNPPQPDVPCDNPPQP
DVPCDNPPQPDVPCDNPPQPDQPDDNPPIPNIPTDWIPNIPTDWIPDIPEKPTTPATTPNI
PA.
**SEQ ID NO: 4 - Eno (*C*. *albicans* Enolase)** 







.
**SEQ ID NO: 5- Bgl2 (*C*. *albicans* Bgl2)** 
MQIKFLTTLATVLTSVAAMGDLAFNLGVKNDDGTCKDVSTFEGDLDFLKSHSKIIKT
YAVSDCNTLQNLGPAAEAEGFQIQLGIWPNDDAHFEAEKEALQNYLPKISVSTIKIFL
VGSEALYREDLTASELASKINEIKDLVKGIKDKNGKSYSSVPVGTVDSWNVLVDGAS
KPAIDAADVVYSNSFSYWQKNSQANASYSLFDDVMQALQTLQTAKGSTDIEFWVGE
TGWPTDGSSYGDSVPSVENAADQWQKGICALRAWGINVAVYEAFDEAWKPDTSGT
SSVEKHWGVWQSDKTLKYSIDCKFN.
**SEQ ID NO: 6- Fba1 (*C*. *albicans* Fba1)** 
MAPPAVLSKSGVIYGKDVKDLFDYAQEKGFAIPAINVTSSSTVVAALEAARDNKAPII
LQTSQGGAAYFAGKGVDNKDQAASIAGSIAAAHYIRAIAPTYGIPVVLHTDHCAKKL
LPWFDGMLKADEEFFAKTGTPLFSSHMLDLSEETDDENIATCAKYFERMAKMGQWL
EMEIGITGGEEDGVNNEHVEKDALYTSPETVFAVYESLHKISPNFSIAAAFGNVHGVY
KPGNVQLRPEILGDHQVYAKKQIGTDAKHPLYLVFHGGSGSTQEEFNTAIKNGVVKV
 
NLDTDCQYAYLTGIRDYVTNKIEYLKAPVGNPEGADKPNKKYFDPRVWVREGEKTM
SKRIAEALDIFHTKGQL.
**SEQ ID NO: 7- Pgk1 (synthetic fragment 1-238 from *Candida albicans* Pgk1)** 
MSLSNKLSVKDLDVAGKRVFIRVDFNVPLDGKTITNNQRIVAALPTIKYVEEHKPKYI
VLASHLGRPNGERNDKYSLAPVATELEKLLGQKVTFLNDCVGPEVTKAVENAKDGE
IFLLENLRYHIEEEGSSKDKDGKKVKADPEAVKKFRQELTSLADVYINDAFGTAHRA
HSSMVGLEVPQRAAGFLMSKELEYFAKALENPERPFLAILGGAKVSDKIQLIDNLLDK
VDMLIVGGG.
**SEQ ID NO: 8- Set1 (synthetic fragment 1-208 from *Candida albicans* Set1)** 
MSYNNRSGGGASGGYSRRGYHGSHRGGYRTGRSKYPEDRYLVGGMLSLNKGSHYE
SSDNRYIPNEIGSKSPENRSHRSSTKDGRTPSGLSTPLSSSDKVSTPISIESINGSDRNTG
VNNKDSEFPKLSHHSDFTSTIPFSRSINPQKNFMVINDSHTPKTDKGIQSKKIRYNGEG
VNHVSDPRIAQSNSNLQKPTKKTKKTPYKQLPQ.
**SEQ ID NO: 9- Cfl91 (*Candida albicans* Cfl91)** 
MKLLPLSFSFLLFTFTFAKQLPVFDGKTYQSYHSRNVYACRMHIHKTIKECSTTDIED
YGCMCTNKDAISTFIQCYAYFQKNSTQAIDFFVKYCDNYGNITTNIDQITDAYEDFLN
NGSDTTTITNINNNNNTTNNTTSNHLDFDYSKYAYGLEASIDQFLTNWQRSFFYGMA
MSSYWGFVLLFGTISNWTQYLFPGLMTDLTGPFSNWWRKNLSLPATFGTKKTQEYT
FWFKYLQGYIPSRLESIINSGFCLVVIIVLSINTYYSVGNEIIFTNYVAQLRYLANRTGIN
STILMPFLVLFAGRNNILQWITRWNYGVFMMFHRFIGRMIFLFVVLHAVTFSIALGVD
YYPLMSFPFMIWGTISTVAGGIIMVQASLYVRRRWYEVFLGLHILLALFFMIGGWVHI
SYLGYEWWVFVAIGLWGIDRIVRVLRMISFGFPEADITLMSDETLKIKIPRPDYWEPIP
GGHIFITFLRKDCFWQSHPFTFAPSVENSNTIVLYTKVKQGVTRKLYNYLVTCPGRRA
KITVGVEGPYGECCAAGKSQTAVFIAGGNGVPGMFSEIYDLAKRSTPDSQKRLKLYW
TIREYRSIHWLYEELNELKKFHNVEVTVFVTKPDSNTYINELSSRVISGKCNSTENFDG
ATTATTSLYEKASFDDKATIVVTNRDITSKYDPNSVKDAIIAQLSHVHFREGRPKINTII
KEEIEDAQGSICFVTCGHPAMVDDIRAEVVGTIGKYKEKRIDYYEQLIGWA.
**SEQ ID NO: 10- Hwp1.3 fragment 1 (synthetic)** QEPCDYPQQPQEPCDYPQQP.
**SEQ ID NO: 11- Eno fragment 1 (synthetic)** DSRGNPTVEVDFTT
**SEQ ID NO: 12- Fba1 fragment 1 (synthetic)** YGKDVKDLFDYAQE
**SEQ ID NO: 13- Pgk1 fragment 1 (synthetic)** VPLDGKTITNNQRI

## Claims

1. A lateral flow immunoassay device for qualitative or quantitative detection of Candida infection in a sample selected from a whole blood, serum, plasma and a bronchoalveolar lavage (BAL) sample comprising a backing support and, on said backing support, a capillary flow array and a wicking pad, wherein the capillary flow array comprises **i)** a sample receiving pad located at one end of the backing support and having pores so as to receive the sample; **ii)** a conjugate release pad being distinct from the sample receiving pad or included to the sample receiving pad and being in capillary contact with the sample receiving pad and being impregnated with at least one detection reagent comprising a first binder specific and binding to the anti-Hwp1 antibody analyte conjugated to a first label moiety; **iii)** a detection pad being distinct from the conjugate release pad and being in capillary contact with the conjugate release pad and comprising a detection membrane, a capture test reagent array comprising at least one reagent test line and a capture control reagent array comprising one control reagent line, said capture reagent and control reagent arrays being immobilized on the detection membrane and **iv)** a wicking membrane at the other end of the backing support and being in capillary contact with the detection pad, wherein said at least one reagent test line comprises a Hwp1.3 protein and said control reagent line comprises a control reagent having a specific affinity for the first binder, wherein a Hwp1.3 protein consists of SEQ ID NO: 1.

2. A lateral flow immunoassay device according to claim 1 wherein the conjugate release pad comprises a detection reagent support wherein the said at least said one detection reagent is bound in a capillary releasable manner, receives the sample from the sample receiving pad and releases the detection reagent from its detection reagent support and a first immuno-complex is formed when the anti-Hwp1 antibody analyte is present in the sample and is combined with the detection reagent, the said first immuno-complex then migrates to the detection pad where it binds to the said at least one reagent test line comprising the Hwp1.3 protein.

3. A lateral flow immunoassay device according to claim 1 or 2 wherein the detection pad further comprises more than one reagent test line in the capture test reagent array.

4. A lateral flow immunoassay device according to any one of claims 1 to 3 wherein the capture test reagent array further comprises at least one Candida cell surface protein, for example selected from Eno (enolase protein), Bgl2 (β-glucosidase (Bgl2) protein), fructose-bisphosphate aldolase (Fba1), Pgk1 (phosphoglycerate kinase), Set1 (Lysine histone methyltransferase) and Cfl91 (Protein similar to ferric reductase Fre10p) or a combination thereof.

5. A lateral flow immunoassay device according to any one of claims 1 to 4, wherein the detection reagent support of the conjugate release pad is non-woven glass fiber.

6. A lateral flow immunoassay device according to any one of claims 1 to 5, wherein the detection reagent is an anti-human antibody such as rabbit or mouse anti-human IgG or IgA (first binder specific and binding to the antibody analyte) conjugated to colored or fluorescent particles (first label moiety).

7. A lateral flow immunoassay device according to any one of claims 1 to 6, wherein the label moiety is selected from a gold nanoparticle, a latex or cellulose particle, a fluorescent label and other colloidal metals and magnetic particles.

8. A lateral flow immunoassay device according to any one of claims 1 to 7, wherein the detection membrane is a membrane of nitrocellulose membrane or cellulose.

9. A lateral flow immunoassay device according to any one of claims 1 to 8, wherein the capture reagents are immobilized across the detection membrane in two capture reagent arrays, test and control reagents, respectively and each of the capture reagent arrays comprise one or more lines.

10. A lateral flow immunoassay device according to any one of claims 1 to 9, wherein it is inserted into a rigid housing, such as a plastic cassette, protecting the capillary flow array and wicking pad and having an opening over the sample receiving pad for loading the sample on to said sample receiving pad and one opening over the capture reagent array in order to read and interpret the result of the test.

11. A method for measuring the content of anti-Hwp1 antibodies in a sample, said method comprising:
a) Providing a previously obtained sample selected from whole blood, serum, plasma and bronchoalveolar
lavage (BAL) potentially contaminated with Candida spp pure or diluted in aqueous solution;
b) Subjecting the said sample to a lateral flow immunoassay comprising **i)** a conjugate release pad comprising detection reagents for said antibodies and **ii)** a detection pad comprising a detection membrane where at least Hwp1.3 protein is bound;
c) Detecting the presence or absence of a detection line on the detection membrane at the location where the said least Hwp1.3 protein is bound, the presence of said detection line being indicative of the presence of anti-Hwp1 antibodies, wherein a Hwp 1.3 protein consists of SEQ ID NO: 1.

12. A method according to claim 11 wherein at least one Candida cell surface protein selected from Eno, Bgl2, Fba1, Pgk1, Set1 and Cfl91 or a combination thereof is further bound to the detection membrane of said lateral flow immunoassay and the detection of the presence of IgG or IgA antibodies to this said at least one Candida cell surface protein is further carried out under step c).

13. A method according to claim 11 wherein at least one Candida cell surface protein selected from Eno, Fba1 and Bgl2 is further bound to the detection membrane of said lateral flow immunoassay and the detection of the presence of IgG or IgA antibodies to this said at least one Candida cell surface protein is further carried out under step c).

14. A kit for qualitative or quantitative detection of Candida infection in a sample, said kit comprising at least one lateral flow immunoassay device according to any one of claims 1 to 10.

15. A kit according to claim 14 further comprising at least one of the following:
- a blood collection device;
- instructions for use;
- a disinfection pad;
- an adhesive plaster;
- a data reader for reading the assay results and optionally transmitting to a database.

## Patentansprüche

1. Immunassay-Vorrichtung mit lateralem Durchfluss zum qualitativen oder quantitativen Nachweis von Candida-Infektion in einer Probe, die ausgewählt ist aus einer Vollblut-, Serum-, Plasma- und einer bronchoalveolären Lavage (BAL) Probe, umfassend eine Trägerunterlage und, auf der Trägerunterlage, eine Kapillardurchflussanordnung und ein Saugpad, wobei die Kapillardurchflussanordnung umfasst: **i)** ein Probenaufnahmepad, das sich an einem Ende der Trägerunterlage befindet und Poren aufweist, um die Probe aufzunehmen; **ii)** ein Konjugat-Freisetzungspad, das sich von dem Probenaufnahmepad unterscheidet oder in dem Probenaufnahmepad enthalten ist und sich in Kapillarkontakt mit dem Probenaufnahmepad befindet und mit mindestens einem Nachweisreagenz imprägniert ist, das einen ersten Binder umfasst, der spezifisch ist und sich an den anti-Hwp1-Antikörper-Analyt bindet, der an einen ersten Markierungsteil konjugiert ist; **iii)** ein Nachweispad, das sich von dem Konjugat-Freisetzungspad unterscheidet und sich in Kapillarkontakt mit dem Konjugat-Freisetzungspad befindet und eine Nachweismembran, eine Einfangtestreagenz-Anordnung, die mindestens eine Reagenztestlinie umfasst, und eine Einfangkontrollreagenz-Anordnung umfasst, die eine Kontrollreagenzlinie umfasst, wobei die Einfangreagenz- und die Kontrollreagenz-Anordnung auf der Nachweismembran immobilisiert sind, und **iv)** eine Saugmembran am anderen Ende der Trägerunterlage und die mit dem Nachweispad in Kapillarkontakt ist, wobei die mindestens eine Reagenztestlinie ein Hwp1.3-Protein umfasst und die Kontrollreagenzlinie ein Kontrollreagenz umfasst, das eine spezifische Affinität für den ersten Binder aufweist, wobei ein Hwp1.3-Protein aus der SEQ ID NR.: 1 besteht.

2. Immunassay-Vorrichtung mit lateralem Durchfluss nach Anspruch 1, wobei das Konjugat-Freisetzungspad einen Nachweisreagenzträger umfasst, wobei das mindestens eine Nachweisreagenz auf eine kapillar freisetzbare Weise gebunden ist, die Probe von dem Probenaufnahmepad aufnimmt und das Nachweisreagenz von seinem Nachweisreagenzträger freisetzt und ein erster Immunkomplex gebildet wird, wenn der anti-Hwp1-Antikörperanalyt in der Probe vorhanden ist und mit dem Detektionsreagenz kombiniert wird, der erste Immunkomplex dann zum Nachweispad migriert, wo er sich an die mindestens eine Reagenztestlinie bindet, die das Hwp1.3-Protein umfasst.

3. Immunassay-Vorrichtung mit lateralem Durchfluss nach Anspruch 1 oder 2, wobei das Detektionspad ferner mehr als eine Reagenztestlinie in der Einfangtestreagenz-Anordnung umfasst.

4. Immunassay-Vorrichtung mit lateralem Durchfluss nach einem der Ansprüche 1 bis 3, wobei die Einfangtestreagenz-Anordnung ferner mindestens ein Candida-Zellen-Oberflächenprotein umfasst, das zum Beispiel ausgewählt ist aus Eno (Enolase-Protein), Bgl2 (β-Glucosidase (Bgl2) Protein), Fructose-Bisphosphat-Aldolase (Fba1), Pgk1 (Phosphoglyceratkinase), Set1 (Lysin-Histon-Methyltransferase) und Cfl91 (Protein, das Eisenreduktase Fre10p ähnlich ist) oder einer Kombination davon.

5. Immunassay-Vorrichtung mit lateralem Durchfluss nach einem der Ansprüche 1 bis 4, wobei der Nachweisreagenzträger des Konjugat-Freisetzungspads ein Glasfaservlies ist.

6. Immunassay-Vorrichtung mit lateralem Durchfluss nach einem der Ansprüche 1 bis 5, wobei das Detektionsreagenz ein Anti-Human-Antikörper ist, wie beispielsweise ein Hase- oder Maus-Anti-Human IgG oder IgA (erster Binder spezifisch und bindet sich an den Antikörperanalyt), der an farbige oder fluoreszente Partikel (erster Markierungsteil) konjugiert ist.

7. Immunassay-Vorrichtung mit lateralem Durchfluss nach einem der Ansprüche 1 bis 6, wobei der Markierungsteil ausgewählt ist aus einem Gold-Nanopartikel, einem Latex- oder Cellulose-Partikel, einer Fluoreszenzmarkierung und anderen kolloidalen Metallen und magnetischen Partikeln.

8. Immunassay-Vorrichtung mit lateralem Durchfluss nach einem der Ansprüche 1 bis 7, wobei die Nachweismembran eine Membran aus Nitrocellulose-Membran oder Cellulose ist.

9. Immunassay-Vorrichtung mit lateralem Durchfluss nach einem der Ansprüche 1 bis 8, wobei die Einfangreagenzien durch die Nachweismembran hindurch in zwei EinfangreagenzAnordnungen, Test- beziehungsweise Kontrollreagenzien, immobilisiert sind und jede der Einfangreagenzanordnungen eine oder mehrere Linien umfasst.

10. Immunassay-Vorrichtung mit lateralem Durchfluss nach einem der Ansprüche 1 bis 9, wobei sie in ein starres Gehäuse, wie beispielsweise eine Kunststoffkassette, eingeführt ist, das die Kapillardurchfluss-Anordnung und das Saugpad schützt und eine Öffnung über dem Probenaufnahmepad zum Laden der Probe auf das Probenaufnahmepad und eine Öffnung über der Einfangreagenz-Anordnung zum Lesen und Interpretieren des Ergebnisses des Tests aufweist.

11. Verfahren zum Messen des Gehalts an anti-Hwp1-Antikörpern in einer Probe, wobei das Verfahren umfasst:
a) Bereitstellen einer vorhergehend erhaltenen Probe, die ausgewählt ist aus Vollblut, Serum, Plasma und branchoalveolärer Lavage (BAL), die potenziell mit Candida spp kontaminiert ist, rein oder verdünnt in wässriger Lösung;
b) Aussetzen der Probe gegenüber einem Immunassay mit lateralem Durchfluss, das umfasst: **i)** ein Konjugat-Freisetzungspad, das Nachweisreagenzien für die Antikörper umfasst, und **ii)** ein Nachweispad, das eine Nachweismembran umfasst, wo mindestens Hwp1.3-Protein gebunden wird;
c) Nachweisen des Vorhandenseins oder Nichtvorhandenseins einer Nachweislinie auf der Detektionsmembran an der Stelle, an der mindestens Hwp1.3-Protein gebunden wird, wobei das Vorhandensein der Nachweislinie das Vorhandensein von anti-Hwp1-Antikörpern angibt, wobei ein Hwp1.3-Protein aus der SEQ ID NR: 1 besteht.

12. Verfahren nach Anspruch 11, wobei mindestens ein Candida-Zellen-Oberflächenprotein, das ausgewählt ist aus Eno, Bgl2, Fba1, Pgk1, Set1 und Cfl91 oder einer Kombination davon, ferner an die Detektionsmembran des Immunassays mit lateralem Durchfluss gebunden wird und der Nachweis des Vorhandenseins von IgG- oder IgA-Antikörpern auf dem mindestens einen Candida-Zellen-Oberflächenprotein ferner unter Schritt c) durchgeführt wird.

13. Verfahren nach Anspruch 11, wobei mindestens ein Candida-Zellen-Oberflächenprotein, das ausgewählt wird aus Eno, Fba1 und Bgl2, ferner an die Nachweismembran des Immunassays mit lateralem Durchfluss gebunden wird und der Nachweis des Vorhandenseins von IgG- oder IgA-Antikörpern gegen dieses mindestens eine Candida-Zellen-Oberflächenprotein ferner unter Schritt c) durchgeführt wird.

14. Kit zum qualitativen oder quantitativen Nachweis von Candida-Infektion in einer Probe, wobei das Kit mindestens eine Immunassay-Vorrichtung mit lateralem Durchfluss nach einem der Ansprüche 1 bis 10 umfasst.

15. Kit nach Anspruch 14, das ferner mindestens eines von Folgendem umfasst:
- eine Blutentnahmevorrichtung;
- Bedienungsanleitungen;
- ein Desinfektionspad;
- ein Heftpflaster;
- ein Datenlesegerät zum Lesen der Assay-Ergebnisse und wahlweise Übertragen an eine Datenbank.

## Revendications

1. Dispositif de dosage immunologique à écoulement latéral pour la détection qualitative ou quantitative d'une infection par Candida dans un échantillon choisi parmi des échantillons de sang entier, de sérum, de plasma et de lavage broncho-alvéolaire (BAL) comprenant un support et, sur ledit support, un réseau d'écoulement capillaire et un tampon à effet de mèche, dans lequel le réseau d'écoulement capillaire comprend i) un tampon de réception d'échantillon situé à une extrémité du support et ayant des pores de façon à recevoir l'échantillon ; ii) un tampon de libération de conjugué qui est distinct du tampon de réception d'échantillon ou inclus dans le tampon de réception d'échantillon et qui est en contact capillaire avec le tampon de réception d'échantillon et qui est imprégné avec au moins un réactif de détection comprenant un premier lieur spécifique et se liant à l'analyte d'anticorps anti-Hwp1 conjugué à un premier fragment marqueur ; iii) un tampon de détection qui est distinct du tampon de libération de conjugué et qui est un contact capillaire avec le tampon de libération de conjugué et qui comprend une membrane de détection, un réseau de réactifs de test de capture comprenant au moins une ligne de test de réactifs et un réseau de réactifs témoins de capture comprenant une ligne de réactifs témoins, lesdits réseaux de réactifs de capture et de réactifs témoins étant immobilisés sur la membrane de détection ; et iv) une membrane à effet de mèche à l'autre extrémité du support qui est en contact capillaire avec le tampon de détection, dans lequel ladite au moins une ligne de test de réactifs comprend une protéine Hwp1.3 et ladite ligne de réactifs témoins comprend un réactif témoin ayant une affinité spécifique pour le premier lieur, dans lequel une protéine Hwp1.3 consiste en la SEQ ID NO : 1.

2. Dispositif de dosage immunologique à écoulement latéral selon la revendication 1, dans lequel le tampon de libération de conjugué comprend un support de réactifs de détection dans lequel ledit au moins un réactif de détection est lié d'une manière libérable par voie capillaire, reçoit l'échantillon depuis le tampon de réception d'échantillon, et libère le réactif de détection de son support de réactifs de détection, et un premier complexe immunologique est formé quand l'analyte d'anticorps anti-Hwp1 est présent dans l'échantillon et est combiné avec le réactif de détection, ledit premier complexe immunologique migre ensuite vers le tampon de détection où il se lie à ladite au moins une ligne de test de réactifs comprenant la protéine Hwp1.3.

3. Dispositif de dosage immunologique à écoulement latéral selon la revendication 1 ou 2, dans lequel le tampon de détection comprend en outre plusieurs lignes de test de réactifs dans le réseau de réactifs de test de capture.

4. Dispositif de dosage immunologique à écoulement latéral selon l'une quelconque des revendications 1 à 3, dans lequel le réseau de réactifs de test de capture comprend en outre au moins une protéine de surface cellulaire de Candida, par exemple choisie parmi Eno (protéine énolase), Bgl2 (protéine β-glucosidase (Bgl2), la fructose-bisphosphate aldolase (Fba1), Pgk1 (phosphoglycérate kinase), Set1 (lysine histone méthyltransférase) et Cfl91 (protéine similaire à la réductase ferrique Fre10p) ou une de leurs combinaisons.

5. Dispositif de dosage immunologique à écoulement latéral selon l'une quelconque des revendications 1 à 4, dans lequel le support de réactifs de détection du tampon de libération de conjugué est en fibres de verre non tissées.

6. Dispositif de dosage immunologique à écoulement latéral selon l'une quelconque des revendications 1 à 5, dans lequel le réactif de détection est un anticorps anti-humain tel qu'une IgG ou IgA anti-humaine de souris (premier lieur spécifique et se liant à l'analyte d'anticorps) conjugué à des particules colorées ou fluorescentes (premier fragment marqueur).

7. Dispositif de dosage immunologique à écoulement latéral selon l'une quelconque des revendications 1 à 6, dans lequel le fragment marqueur est choisi parmi une nanoparticule d'or, une particule de latex ou de cellulose, un marqueur fluorescent et d'autres métaux colloïdaux et particules magnétiques.

8. Dispositif de dosage immunologique à écoulement latéral selon l'une quelconque des revendications 1 à 7, dans lequel la membrane de détection est une membrane de nitrocellulose ou de cellulose.

9. Dispositif de dosage immunologique à écoulement latéral selon l'une quelconque des revendications 1 à 8, dans lequel les réactifs de capture sont immobilisés au sein de la membrane de détection en deux réseaux de réactifs de capture, réactifs de test et témoins, respectivement, et chacun des réseaux de réactifs de capture comprend une ou plusieurs lignes.

10. Dispositif de dosage immunologique à écoulement latéral selon l'une quelconque des revendications 1 à 9, qui est inséré dans un boîtier rigide, tel qu'une cassette plastique, qui protège le réseau d'écoulement capillaire et le tampon à effet de mèche et qui a une ouverture au-dessus du tampon de réception d'échantillon pour charger l'échantillon sur ledit tampon de réception d'échantillon et une ouverture au-dessus du réseau de réactifs de capture afin de lire et d'interpréter le résultat du test.

11. Procédé pour mesurer la teneur d'un échantillon en anticorps anti-Hwp1, ledit procédé comprenant :
a) la fourniture d'un échantillon obtenu au préalable choisi parmi le sang entier, le sérum, le plasma et un lavage broncho-alvéolaire (BAL), potentiellement contaminé par Candida spp., pur ou dilué en solution aqueuse ;
b) la soumission dudit échantillon à un dosage immunologique à écoulement latéral comprenant i) un tampon de libération de conjugué comprenant des réactifs de détection pour lesdits anticorps et ii) un tampon de détection comprenant une membrane de détection où au moins une protéine Hwp1.3 est liée ;
c) la détection de la présence ou de l'absence d'une ligne de détection sur la membrane de détection à l'endroit où ladite au moins une protéine Hwp1.3 est liée, la présence de ladite ligne de détection étant indicative de la présence d'anticorps anti-Hwp1,
dans lequel une protéine Hwp1.3 consiste en la SEQ ID NO : 1.

12. Procédé selon la revendication 11, dans lequel au moins une protéine de surface cellulaire de Candida choisie parmi Eno, Bgl2, Fba1, Pgk1, Set1 et Cfl91, ou une de leurs combinaisons, est en outre liée à la membrane de détection dudit dosage immunologique à écoulement latéral, et la détection de la présence d'anticorps IgG ou IgA à cette dite au moins une protéine de surface cellulaire de Candida est en outre effectuée dans le cadre de l'étape c).

13. Procédé selon la revendication 11, dans lequel au moins une protéine de surface cellulaire de Candida choisie parmi Eno, Fba1 et Bgl2 est en outre liée à la membrane de détection dudit dosage immunologique à écoulement latéral, et la détection de la présence d'anticorps IgG ou IgA à cette dite au moins une protéine de surface cellulaire de Candida est en outre effectuée dans le cadre de l'étape c).

14. Trousse pour la détection qualitative ou quantitative d'une infection par Candida dans un échantillon, ladite trousse comprenant au moins un dispositif de dosage immunologique à écoulement latéral selon l'une quelconque des revendications 1 à 10.

15. Trousse selon la revendication 14, comprenant en outre au moins l'un des suivants :
- un dispositif de collecte de sang ;
- des directives d'utilisation ;
- un tampon de désinfection ;
- un emplâtre adhésif ;
- un lecteur de données pour lire les résultats du dosage et éventuellement les transmettre à une base de données.
